# FASCICULE DE BREVET EUROPEEN

(11) **EP 2 321 240 B1**
(45) Date de publication et mention de la délivrance du brevet: **31.12.2014**
(21) Numéro de dépôt: 09740468.5
(22) Date de dépôt: 18.08.2009
(51) Int. Cl.: C07C 17/25, C07C 21/18

(54) **PROCEDE DE PREPARATION DE COMPOSES FLUORES OLEFINIQUES**
VERFAHREN ZUR HERSTELLUNG FLUORINIERTER OLEFINISCHER VERBINDUNGEN
PROCESS FOR THE PREPARATION OF FLUORINATED OLEFINIC COMPOUNDS

(30) Priorité: 11.09.2008 FR 0856112
(43) Date de publication de la demande: 18.05.2011
(73) Titulaire: Arkema France, 92700 Colombes (FR)
(72) Inventeur: PIGAMO, Anne, F-69340 Francheville (FR); DEVIC, Michel, F-69110 Sainte Foy Les Lyon (FR); WENDLINGER, Laurent, F-69510 Soucieu En Jarrest (FR)
(74) Mandataire: Dang, Doris
(86) Numéro de dépôt international: PCT/FR2009/051594
(87) Numéro de publication internationale: WO 2010/029239

(56) Documents cités:
- WO-A1-2007/056194
- WO-A2-2008/075017
- SIANESI DARIO ET AL: "Fluoro olefins. I. cis- and trans-1,2,3,3,3-Pentafluoropropylene" ANNALI DI CHIMICA, SOCIETA CHIMICA ITALIANA, ROME, IT, vol. 55, no. 8-9, 1 janvier 1965 (1965-01-01), pages 850-861, XP009092725 ISSN: 0003-4592
- KNUNYANTS I L ET AL: "REACTIONS OF FLUORO OLEFINS. ÖCOMMUNICATION 13. CATALYTIC HYDROGENATION OF PERFLUORO OLEFINS" BULLETIN OF THE ACADEMY OF SCIENCES OF THE USSR, DIVISION OFCHEMICAL SCIENCES,, 1 janvier 1960 (1960-01-01), pages 1312-1317, XP000578879 ISSN: 0568-5230 cité dans la demande

## Description

### DOMAINE DE L'INVENTION

L'invention a pour objet un procédé de préparation de composés fluorés oléfiniques. Elle concerne plus particulièrement un procédé de préparation de 1,2,3,3,3-pentafluoropropène (HFO-1225ye) et de 2,3,3,3-tétrafluoro-1-propène (HFO-1234yf).

### ARRIERE-PLAN TECHNOLOGIQUE

Les hydrofluorocarbones (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

Le 1,2,3,3,3- pentafluoropropène (HFO-1225ye) est un intermédiaire de synthèse dans la fabrication du 2,3,3,3-tétrafluoro-1-propène (HFO-1234yf).

La plupart des procédés de fabrication des hydrofluorooléfines font appel à une réaction de déhydrohalogénation. Ainsi, le document WO 03/027051 décrit un procédé de fabrication de fluorooléfines de formule CF₃CY=CXₙHₚ, dans laquelle X représente un atome d'hydrogène ou d'halogène choisi parmi le fluor, le chlore, le brome ou l'iode ; n et p sont des nombres entiers et peuvent indépendamment prendre la valeur zéro, 1 ou 2 à condition que (n + p) = 2, comprenant la mise en contact d'un composé de formule CF₃C(R¹ₐR²_{b})C(R³_{c}R⁴_{d}) avec R¹, R², R³ et R⁴ représentant indépendamment un atome d'hydrogène ou d'halogène choisi parmi le fluor, le chlore, le brome ou l'iode à condition qu'au moins un de R¹, R², R³ et R⁴ est un atome d'halogène et qu'au moins un atome d'hydrogène et un atome d'halogène sont situés sur des atomes de carbone adjacent ; a et b peuvent indépendamment prendre la valeur zéro, 1 ou 2 à condition que (a + b) = 2 ; c et d peuvent indépendamment prendre la valeur zéro, 1, 2 ou 3 à condition que (c + d) = 3, avec au moins un hydroxyde de métal alcalin en présence d'un catalyseur de transfert phase.

Ce document enseigne à l'exemple 2, qu'en l'absence d'un catalyseur de transfert de phase, il n'y a pas de réaction lorsque le 1,1,1,3,3-pentafluoropropane (HFC-245fa) est mis en contact avec une solution aqueuse de 50 % en poids de hydroxyde de potassium (KOH) pendant 24 heures à température ambiante et sous pression.

Ce document enseigne en outre une température de réaction compris entre -20°C et 80°C.

Le document WO 2008/075017 illustre la réaction de déhydrofluoration du 1,1,1,2,3,3-hexafluoropropane (HFC-236ea) en 1,2,3,3,3-pentafluoropropène (HFO-1225ye) à 150°C en présence d'une solution aqueuse de 50 % en poids de KOH. En l'absence d'un catalyseur de transfert de phase, la conversion au bout de 3 heures et demie est de 57,8 % et la sélectivité en HFO-1225ye est de 52,4 % (essai 1). En présence d'un catalyseur de transfert de phase, cette conversion est atteinte au bout de 2,5 heures seulement et la sélectivité est pratiquement inchangée (essai 4). Comme indiqué au tableau 2 de ce document pour accroître la sélectivité en HFO-1225ye, il est nécessaire d'utiliser un solvant organique.

WO 2007/056194 décrit la préparation de HFO-1234yf par déhydrofluoration du 1,1,1,2,3-pentafluoropropane (HFC-245eb) soit avec une solution aqueuse de KOH soit en phase gazeuse en présence d'un catalyseur, notamment sur catalyseur à base de nickel, de carbone ou une combinaison de ceux-ci.

Le document Knunyants et al, Journal of the USSR Academy of Sciences, Chemistry Department, "reactions of fluoro-olefins", report 13., "catalytic hydrogénation of perfluoro-olefins", 1960, décrit de façon distincte diverses réactions chimiques sur des composes fluorés. Ce document décrit la déhydrofluoration du 1,1,1,2,3,3-hexafluoropropane (236ea) par passage au travers d'une suspension de KOH en poudre dans l'éther de dibutyle, pour produire du 1,2,3,3,3-pentafluoropropène-1 (HFO-1225ye) avec un rendement de 60 % seulement. Ce document décrit également la déhydrofluoration de 1,1,1,2,3-pentafluoropropane (HFC-245eb) en 2,3,3,3-tétrafluoro-1-propène (HFO-1234yf) par passage dans une suspension de KOH en poudre dans l'éther de dibutyle avec un rendement de 70 % seulement.

Par ailleurs, la figure 2 à la page 51 du deuxième fascicule du nouveau traité de chimie minérale de P. Pascal, Ed. 1963, montre l'allure des équilibres liquide solide du système eau et hydroxyde de potassium et les mesures sont rassemblées dans le tableau à la page 52.

Le document Sianesi Dario et al (Annali di chimica Societa Chimica Haliana, vol. 55 n° 8-9, 1965, pages 850 - 861) décrit la déhydrafluorination de l'hexafluoropropane, sans préciser l'isomère, par du KOH.

Il a maintenant été trouvé un procédé de fabrication d'un composé de formule (I) CF₃-CF=CHX dans lequel X représente un atome d'hydrogène ou fluor, comprenant au moins une étape de déhydrofluoration d'un composé de formule (II) CF₃-CHF-CHFX, avec une très bonne sélectivité et/ou rendement élevé.

La présente invention a donc pour objet un procédé de fabrication d'un composé de formule (I) CF₃-CF=CHX dans lequel X représente un atome d'hydrogène ou fluor, comprenant au moins une étape de déhydrofluoration d'un composé de formule (II) CF₃-CHF-CHFX, au cours de laquelle ou desquelles le composé de formule (II) est mis en contact avec un mélange constitué d'eau et d'hydroxyde de potassium, dans lequel l'hydroxyde de potassium est présent en quantité comprise entre 58 et 69 % en poids et avantageusement comprise entre 60 et 66 % en poids, ledit mélange étant maintenu à une température comprise entre 125 et 180°C.

De préférence, la température du mélange eau et hydroxyde est comprise entre 145 et 180°C, et avantageusement entre 152 et 165°C.

Selon un mode de réalisation de l'invention, le mélange d'eau et d'hydroxyde peut être obtenu à partir des hydrates de formule KOH. x H₂O, x étant compris entre 1 et 2 inclus.

L'étape de déhydrofluoration peut être mise en oeuvre dans tout type de réacteur connu de l'homme de l'art. On peut utiliser, un réacteur agité, un mélangeur statique, une colonne réactive, une tuyère ou tout simplement faire buller le composé de formule (II) dans ledit mélange d'eau et d'hydroxyde de potassium contenu dans un récipient.

Outre les étapes de déhydrofluoration, le procédé comprend une étape de séparation du composé de formule (I) suivie éventuellement d'une étape de purification.

On peut opérer de façon continue, semi continue, ou discontinue à pression atmosphérique ou sous pression, de préférence inférieure à 2 bars absolus.

La demanderesse a observé que la vitesse de déhydrofluoration du composé de formule (II) est très élevée avec le mélange d'eau et d'hydroxyde tel que décrit précédemment et donc particulièrement intéressante pour mettre en oeuvre le procédé, selon la présente invention, en continu.

Selon un mode de réalisation préféré de l'invention, on introduit en continu le composé de formule (II) et le mélange constitué d'eau et d'hydroxyde tel que défini précédemment dans un réacteur, initialement chargé de ce mélange, maintenu à la température précitée et on soutire en continu de la phase gaz du réacteur un flux comprenant le composé de formule (I). Le fluorure de potassium formé comme sous produit peut être soutiré de façon continue ou discontinue du milieu réactionnel liquide par exemple par filtration. La concentration en eau du milieu réactionnel peut être maintenue constante par évaporation en continu de l'eau formée par la réaction.

Lorsqu'on opère en discontinu ou semi-continu, le ratio molaire KOH / composé de formule (II) mis enjeu, est en général compris entre 2 et 100, de préférence compris entre 3 et 20.

Le procédé convient tout particulièrement à la fabrication du 2,3,3,3-tetrafluoropropène par déhydrofluoration du 1,2,3,3,3-pentafluoropropane.

Il convient également à la fabrication du 1,2,3,3,3-pentafluoropropène par déhydrofluoration du 1,1,1,2,3,3-hexafluoropropane. Le 1,2,3,3,3-pentafluoropropène peut être sous la forme d'isomère Z et/ou E.

La demanderesse a découvert que le sous produit trifluorure de vinyle peut être réduit dans le cas de la déhydrofluoration du HFC-236ea. Ce sous-produit devient négligeable, lorsque la température de déhydrofluoration est comprise entre 125 et 145 °C et le KOH est présent entre 58 et 69 % en poids, de préférence entre 60 et 66 % en poids dans le mélange eau-KOH.

Il peut être avantageux d'utiliser un gaz inerte, de préférence de l'azote ou de l'hydrogene dans l'étape de déhydofluoration.

Selon la conversion recherchée, la réaction de déhydrohalogénation peut être mise en oeuvre en plusieurs étapes, éventuellement à l'aide de plusieurs réacteurs en série, de préférence en deux étapes, éventuellement à l'aide de deux réacteurs en série.

Le procédé selon la présente invention a l'avantage de conduire à des rendements élevés même en l'absence de catalyseur de transfert de phase et/ou de solvant organique.

### PARTIE EXPERIMENTALE

La conversion est définie comme le pourcentage du composé de formule (II) ayant réagi (nombre de moles de composé de formule (II) réagi / nombre de moles de composé de formule (II) introduit).

La sélectivité est définie comme le pourcentage du ratio du nombre de moles de composé de formule (I) ou sous-produit formé / nombre de moles de composé de formule (II) ayant réagi.

Le rendement est défini comme pourcentage du ratio du nombre de moles de composé de formule (I) ou sous-produit formé / nombre de moles de composé de formule (II) introduit:

### Exemple 1

Dans un réacteur cylindrique d'un litre, en acier inox, équipé d'un dispositif de chauffage et de mesure de température du milieu réactionnel, on introduit 1005 g d'un mélange eau et KOH dans lequel le KOH est présent à 66 % en poids. A l'aide d'un tube plongeur, on fait buller dans le milieu maintenu à 135°C du HFC-236ea avec un débit de 150 g/h et de l'hydrogène avec un débit de 12 Nl/h. Les produits gazeux sortent du réacteur par un orifice situé sur le couvercle et sont séchés par une colonne de tamis moléculaire, puis recueillis dans un piège en acier inox refroidi par de l'azote liquide. On arrête la réaction après introduction de 48,3 g de HFC-236ea et on obtient :
- une conversion de 92,7 % en HFC-236ea
- une sélectivité de 99 % en HFO-1225ye (E + Z)
- un rendement de 89,9 % en HFO-1225yeE.

La concentration molaire du CF₂=CFH dans le liquide recueilli dans le piège à la sortie du réacteur est de 0,02 % .

### Exemple 2

On utilise deux réacteurs en série, du même type que celui de l'exemple 1 à l'exception du premier réacteur qui a un volume de 4 litres, le flux gazeux issu du premier réacteur alimente le deuxième réacteur. On introduit dans le premier réacteur 4380 g d'un mélange d'eau-KOH avec le KOH présent à 66 % en poids et dans le deuxième réacteur 1200 g d'un mélange d'eau-KOH avec le KOH présent à 70 % en poids, la température du milieu est maintenue à 160°C. On introduit du HFC-236ea dans le premier réacteur avec un débit de 165 g/h et on arrête la réaction après introduction de 1100 g de HFC-236ea dans le premier réacteur.

On obtient une conversion de 99,3 % de HFC-236ea, une sélectivité en 1225ye (E+Z) de 98 %. La concentration molaire du CF₂=CFH dans le liquide recueilli dans le piège à la sortie du réacteur est de 0,19 %.

### Exemple 3 (non conforme à l'invention)

On opère avec l'appareillage de l'exemple 1. On introduit dans le réacteur 1055 g d'un mélange eau et KOH dans lequel le KOH est présent à 86 % en poids. On introduit dans le milieu maintenu à 165°C, du HFC-236ea avec un débit de 150 g/h et on arrête la réaction après introduction de 1000 g de HFC-236ea.

On obtient une conversion de 98,2 % de HFC-236ea, une sélectivité en HFO-1225ye (E+Z) de 95 %. La concentration molaire du CF₂=CFH dans le liquide recueilli dans le piège à la sortie du réacteur est de 1 %.

### Exemple 4 (non conforme à l'invention)

On opère avec l'appareillage de l'exemple 1. On introduit dans le réacteur 1000 g d'un mélange d'eau et KOH, le KOH étant présent à 75 % en poids. On introduit dans le milieu maintenu à 160°C, du HFC-245eb avec un débit de 150 g/h et on arrête la réaction après introduction de 276 g de HFC-245eb.

On obtient une conversion de 83,2 % de HFC-245eb, une sélectivité de 99 % en HFO-1234yf. La concentration molaire de tetrafluoropropane dans le liquide recueilli dans le piège à la sortie du réacteur est de 1,5 %.

## Revendications

1. Procédé de fabrication d'un composé de formule (I) CF₃-CF=CHX, dans lequel X représente un atome d'hydrogène ou fluor, comprenant au moins une étape de déhydrofluoration d'un composé de formule (II) CF₃-CHF-CHFX (X ayant la même signification que dans la formule (I)), au cours de laquelle ou desquelles le composé de formule (II) est mis en contact avec un mélange constitué d'eau et d'hydroxyde de potassium, dans lequel l'hydroxyde de potassium est présent en quantité comprise entre 58 et 69 % en poids et avantageusement comprise entre 60 et 66 % en poids, ledit mélange étant maintenu à une température comprise entre 125 et 180°C.

2. Procédé selon la revendication 1 **caractérisé en ce que** X dans les formules (I) et (II) représente un atome d'hydrogène.

3. Procédé selon la revendication 1 **caractérisé en ce que** X dans les formules (I) et (II) représente un atome de fluor.

4. Procédé selon l'une quelconque des revendications précédentes **caractérisé en ce que** l'on opère en continu.

5. Procédé selon l'une quelconque des revendications précédentes **caractérisé en ce qu'**il comprend au moins deux étapes de déhydrofluoration.

6. Procédé selon l'une quelconque des revendications précédentes **caractérisé en ce que** l'on opère en l'absence de solvant organique et/ou catalyseur de transfert de phase.

7. Procédé selon l'une quelconque des revendications précédentes **caractérisé en ce que** le mélange eau et hydroxyde est obtenu à partir des hydrates de formule KOH. x H₂O, x étant compris entre 1 et 2 inclus.

8. Procédé selon l'une quelconque des revendications précédentes **caractérisé en ce qu'**il comprend une étape de séparation du composé de formule (I) et éventuellement une étape de purification.

9. Procédé selon l'une quelconque des revendications précédentes **caractérisé en ce que** la température du mélange est comprise entre 145 et 180°C, de préférence entre 152 et 165°C.

## Patentansprüche

1. Verfahren zur Herstellung einer Verbindung der Formel (I) CF₃-CF=CHX, worin X für ein Wasserstoff- oder Fluoratom steht, umfassend mindestens einen Schritt der Dehydrofluorierung einer Verbindung der Formel (II) CF₃-CHF-CHFX (wobei X die gleiche Bedeutung wie in Formel (I) besitzt), bei dem bzw. denen die Verbindung der Formel (II) mit einer aus Wasser und Kaliumhydroxid bestehenden Mischung, in der das Kaliumhydroxid in einer Menge zwischen 58 und 69 Gew.-% und vorzugsweise zwischen 60 und 66 Gew.-% vorliegt, in Berührung gebracht wird, wobei die Mischung bei einer Temperatur zwischen 125 und 180°C gehalten wird.

2. Verfahren nach Anspruch 1, **dadurch gekennzeichnet, dass** X in den Formeln (I) und (II) für ein Wasserstoffatom steht.

3. Verfahren nach Anspruch 1, **dadurch gekennzeichnet, dass** X in den Formeln (I) und (II) für ein Fluoratom steht.

4. Verfahren nach einem der vorhergehenden Ansprüche, **dadurch gekennzeichnet, dass** man es kontinuierlich betreibt.

5. Verfahren nach einem der vorhergehenden Ansprüche, **dadurch gekennzeichnet, dass** es mindestens zwei Dehydrofluorierungsschritte umfasst.

6. Verfahren nach einem der vorhergehenden Ansprüche, **dadurch gekennzeichnet, dass** man es in Abwesenheit von organischem Lösungsmittel und/oder Phasentransferkatalysator betreibt.

7. Verfahren nach einem der vorhergehenden Ansprüche, **dadurch gekennzeichnet, dass** die Mischung von Wasser und Hydroxid aus Hydraten der Formel KOH. x H₂O, wobei x zwischen 1 und 2 inklusive liegt, erhalten wird.

8. Verfahren nach einem der vorhergehenden Ansprüche, **dadurch gekennzeichnet, dass** es einen Schritt der Abtrennung der Verbindung der Formel (I) und gegebenenfalls einen Reinigungsschritt umfasst.

9. Verfahren nach einem der vorhergehenden Ansprüche, **dadurch gekennzeichnet, dass** die Temperatur der Mischung zwischen 145 und 180°C, vorzugsweise zwischen 152 und 165°C, liegt.

## Claims

1. Process for the manufacture of a compound of formula (I) CF₃-CF=CHX in which X represents a hydrogen or fluorine atom, comprising at least one stage of dehydrofluorination of a compound of formula (II) CF₃-CHF-CHFX (X having the same meaning as in the formula (I)) during which the compound of formula (II) is brought into contact with a mixture composed of water and of potassium hydroxide in which the potassium hydroxide is present in an amount of between 58 and 69% by weight and advantageously of between 60 and 66% by weight, the said mixture being maintained at a temperature of between 125 and 180°C.

2. Process according to Claim 1, **characterized in that** X in the formulae (I) and (II) represents a hydrogen atom.

3. Process according to Claim 1, **characterized in that** X in the formulae (I) and (II) represents a fluorine atom.

4. Process according to any one of the preceding claims, **characterized in that** it is carried out continuously.

5. Process according to any one of the preceding claims, **characterized in that** it comprises at least two dehydrofluorination stages.

6. Process according to any one of the preceding claims, **characterized in that** it comprises at least two dehydrofluorination stages.

7. Process according to any one of the preceding claims, **characterized in that** the water and hydroxide mixture is obtained from hydrates of formula KOH · × H₂O, x being between 1 and 2 inclusive.

8. Process according to any one of the preceding claims, **characterized in that** it comprises a stage of separation of the compound of formula (I) and optionally a purification stage.

9. Process according to any one of the preceding claims, **characterized in that** the temperature of the mixture is between 145 and 180°C, preferably between 152 and 165°C.
